**Europäisches Patentamt**

**European Patent Office**

**Office européen des brevets**

(19)

(11) Veröffentlichungsnummer: **0 236 839**
**B1**

# (12) EUROPÄISCHE PATENTSCHRIFT

(45) Veröffentlichungstag der Patentschrift:
**24.10.90**

(21) Anmeldenummer: **87102582.1**

(22) Anmeldetag: **24.02.87**

(51) Int. Cl.⁵: **C07C 209/82**
// C07C209/32

(54) **Verfahren zur Aufarbeitung wässriger Aminlösungen.**

(30) Priorität: **08.03.86 DE 3607665**

(43) Veröffentlichungstag der Anmeldung:
**16.09.87 Patentblatt 87/38**

(45) Bekanntmachung des Hinweises auf die Patenterteilung:
**24.10.90 Patentblatt 90/43**

(84) Benannte Vertragsstaaten:
**BE DE ES FR GB IT NL**

(56) Entgegenhaltungen:
**US-A-3 421 983**
**US-A-3 428 531**

(73) Patentinhaber: **BAYER AG,**
**D-5090 Leverkusen 1 Bayerwerk(DE)**

(72) Erfinder: **Beckhaus, Heiko, Dr., Schulstrasse 20,**
**D-2212 Brunsbüttel(DE)**
Erfinder: **Witt, Harro, Dr., Möhlenbag 2,**
**D-2224 Kuden(DE)**
Erfinder: **Becher, Dieter, Dr., Moltkestrasse 8,**
**D-4047 Dormagen(DE)**
Erfinder: **Dallmeyer, Hermann, Dr., Im Alten Driesch 22,**
**D-5068 Odenthal(DE)**
Erfinder: **Zarnack, Uwe J., Dr., Ulitzhörn 2,**
**D-2212 Brunsbüttel(DE)**

## Beschreibung

Die Erfindung betrifft ein neues Verfahren zur destillativen Aufarbeitung von wäßrigen Aminlösungen, wie sie bei der Hydrierung von technischen Dinitroaromaten anfallen, wobei in einem Arbeitsschritt sowohl von wasserdampfflüchtigen Verunreinigungen und von Wasser weitgehend befreites Diamin als auch von wasserdampfflüchtigen organischen Verunreinigungen weitgehend befreites Wasser anfallen.

Es ist bekannt (vgl. z.B. DE-OSen 1 542 544, 1 947 851, 2 106 644, 2 135 154, 2 214 056, 2 456 308, BE-PSen 631 964, 661 047, 661 946, FR-PS 1 359 438 oder GB-PS 768 111), daß man aromatische Diamine durch katalytische Hydrierung von den entsprechenden aromatischen Dinitroverbindungen herstellen kann. Die Hydrierung kann unter Mitverwendung von Lösungsmitteln wie beispielsweise niedrig siedenden Alkoholen wie Methanol, Ethanol oder Isopropanol aber auch ohne solche Fremdlösungsmittel erfolgen. Die Hydrierung kann mit Hilfe von im Reaktionsgemisch dispergierten Katalysatoren durchgeführt werden, die dann durch Sedimentation oder Filtration abgetrennt und gegebenenfalls in den Prozeß zurückgeführt werden.

Bislang erfolgte die Aufarbeitung des Reaktionsgemisches dergestalt, daß man ein nach Abtrennung des gegebenenfalls mitverwendeten Hilfslösungsmittels vorliegendes Gemisch aus aromatischen Diaminen und Reaktionswasser zunächst kontinuierlich unter Normaldruck in einer Destillationskolonne vom Wasser und das als Destillationsrückstand anfallende Diamin gegebenenfalls in weiteren Verfahrensschritten von anhaftendem Wasser und von gegebenenfalls noch vorliegenden organischen Verunreinigungen befreit. Bei dieser Arbeitsweise entstehen als Destillate stets Gemische von Wasser mit wasserdampfflüchtigen organischen Nebenprodukten, wie sie bei der Hydrierung der Dinitroaromaten anfallen. Es handelt sich bei diesen Nebenprodukten beispielsweise um aromatische oder cycloaliphatische Monoamine und/oder um cycloaliphatische Alkohole, d.h. beispielsweise im Falle der Herstellung von Diaminotoluol um Toluidine, Perhydrotoluidine und/oder Methylcyclohexanole.

Diese wasserdampfflüchtigen Nebenprodukte bewirken, daß das über Kopf abdestillierte Wasser stark mit diesen Verbindungen belastet ist und nur mit großem Aufwand, beispielsweise über eine biologische Kläranlage entsorgt werden kann.

Es war die der Erfindung zugrundeliegende Aufgabe, ein neues Verfahren zur Aufarbeitung von derartigen wässrigen Aminlösungen zur Verfügung zu stellen, bei welchem ein weit weniger mit organischen Verunreinigungen belastetes Abwasser anfällt.

Diese Aufgabe konnte mit dem nachstehend näher erläuterten erfindungsgemäßen Verfahren gelöst werden. Außerdem gestattet das erfindungsgemäße Verfahren eine weitgehende Wiedergewinnung der zur Verdampfung des Wassers aufgewandten Energie durch Nutzung der Kondensationswärme der Brüden.

Gegenstand der Erfindung ist ein Verfahren zur Aufarbeitung von wässrigen Aminlösungen, wie sie bei der katalytischen Hydrierung von aromatischen Dinitroverbindungen anfallen, und die neben den entsprechenden aromatischen Diaminoverbindungen wasserdampfflüchtige organische Verbindungen als Produkte von Nebenreaktionen enthalten, durch destillative Abtrennung des Wassers zusammen mit den wasserdampfflüchtigen organischen Nebenprodukten in einer Destillationskolonne, dadurch gekennzeichnet, daß man

a) die Brüden der Destillationskolonne kondensiert, die hierbei anfallende flüssige Phase über eine Trennapparatur führt, in welcher dem Brüdenkondensat wasserdampfflüchtige organische Nebenprodukte als organische Phase entnommen werden,

b) die die Trennapparatur verlassende, wässrige Phase auf den Kopf der Destillationskolonne zurückführt,

c) der Destillationskolonne des weitgehend von wasserdampfflüchtigen organischen Verunreinigungen befreite Wasser über einen Seitenstrom entnimmt und

d) die von Wasser und wasserdampfflüchtigen Verunreinigungen weitgehend befreiten Diamine als Sumpfprodukt gewinnt.

Ausgangsmaterialien für das erfindungsgemäße Verfahren sind wäßrige Aminlösungen, wie sie bei der Hydrierung von Dinitroaromaten anfallen. Besonders bevorzugt werden beim erfindungsgemäßen Verfahren wäßrige Aminlösungen eingesetzt, wie sie bei der Hydrierung von technischen Dinitrotoluolen anfallen, und die von gegebenenfalls bei der Hydrierung mitverwendetem Hilfslösungsmittel, wie den oben genannten einfachen Alkoholen, vorab destillativ befreit worden sind. Bei diesen Lösungen handelt es sich im allgemeinen um ca. 50-70-gew.-%ige, vorzugsweise 55-65-gew.-%ige Lösungen von Diaminotoluolen in Wasser, wobei diese Lösungen im allgemeinen bis zu 5 %, vorzugsweise 500 - 3000 ppm (Gewicht) an wasserdampfflüchtigen Verunreinigungen der oben beispielhaft genannten Art enthalten. Bei diesen Diaminen handelt es sich z.B. um reines 2,4-Diaminotoluol oder um dessen technische Gemische mit bis zu 40 Gew.-%, bezogen auf Gesamtgewicht, an 2,6-Diaminotoluol und gegebenenfalls bis zu 5 Gew.%, bezogen auf Gesamtgemisch, an anderen isomeren Diaminotoluolen, wobei sich die Prozentsätze jeweils zu 100 ergänzen.

Zur Durchführung des erfindungsgemäßen Verfahrens wird eine Destillationskolonne, beispielsweise eine Glockenbodenkolonne oder Fülkörperkolonne verwendet. Vorzugsweise weist die Kolonne 12 bis 40 insbesondere 20 bis 40 theoretische Böden auf. Die Kolonne wird bei einer Sumpftemperatur von 120 bis 210, vorzugsweise 140 bis 180°C und unter einem Druck (am Kopf der Kolonne) von mindestens 1, vorzugsweise 1,5 bis 6 bar abs. betrieben, wobei diese Parameter selbstverständlich von der Natur der aufzuarbeitenden Gemische und von der angestrebten Brüdentemperatur abhängen. So kann die Kondensationswärme der Brüden bei Arbeiten unter Überdruck leicht so eingestellt werden, daß sie beispielsweise zur Verdampfung von Lösungsmitteln, zur Erwärmung von Produktströmen oder zur Erzeugung von Prozeßdampf genutzt werden kann.

Die Einspeisung der destillativ aufzuarbeitenden Lösungen erfolgt im allgemeinen oberhalb des, beispielsweise mittels eines Umlaufverdampfers beheizten Sumpfs, d.h. mindestens auf den zweiten, vorzugsweise an einer Stelle zwischen dem dritten und achten theoretischen Boden. Die Brüden werden bei der Durchführung des erfindungsgemäßen Verfahrens vollständig kondensiert und nach Abtrennung einer organischen Phase auf den Kopf der Kolonne zurückgeführt. Die Destillatentnahme erfolgt mittels eines Entnahmebodens über einen Seitenstrom, der mindestens 4, vorzugsweise 5 bis 15 theoretische Böden unterhalb des Kopfs der Kolonne und mindestens 8, vorzugsweise 12 bis 25 theoretische Böden oberhalb des Sumpfs der Kolonne angeordnet ist. Hierbei liegt das Volumenverhältnis von Rücklauf (unterhalb der Entnahmestelle) zu Entnahme bei mindestens 0,3, vorzugsweise 0,4 bis 0,6.

Bei einem kontinuierlichen Betrieb der Destillationskolonne unter diesen Bedingungen reichern sich die wasserdampfflüchtigen organischen Nebenprodukte überraschenderweise im Kolonnenkopf an und können, sobald ihre Konzentration über die Löslichkeitsgrenze in Wasser ansteigt, dem Brüdenkondensat mittels einer Trennapparatur entnommen werden.

Bei dieser Trennapparatur, die zwischen dem Brüdenkondensator und dem Kolonnenkopf in der Rücklaufleitung instal liert ist, kann es sich beispielsweise um einen Phasenabscheider handeln. Es ist jedoch auch möglich, die im Brüdenkondensat vorliegenden organischen Verunreinigungen ganz oder teilweise durch Sedimentieren, Zentrifugieren, Adsorbieren an Adsorbentien oder Extrahieren aus dem Brüdenkondensat zu entfernen, bevor dieses in den Kolonnenkopf zurückgeführt wird. Das über den Seitenstrom abgezogene Wasser ist nur noch sehr geringfügig durch organische Verbindungen verunreinigt. Während beispielsweise bei der einfachen destillativen Abtrennung des Wassers aus wässrigen Amingemischen, wie sie bei der Hydrierung von technischen Dinitrotoluolen anfallen, das als Kondensat anfallende Wasser ein Gehalt von bis zu 5, vorzugsweise 0,2 bis 5 und besonders bevorzugt 2 - 5 Gew.-% an organischen Nebenprodukten enthält, weist das beim erfindungsgemäßen Verfahren aus analogen Ausgangsgemischen in Form des Seitenstroms erhaltene Wasser lediglich einen Gehalt an derartigen Verunreinigungen von 10 bis 500 ppm (Gewicht) auf.

Beim erfindungsgemäßen Verfahren werden die vom Wasser weitgehend befreiten aromatischen Diamine kontinuierlich als Sumpfprodukt ausgetragen. Die am Kopf der Kolonne kontinuierlich abgetrennten Organika können als chemische Rohstoffe weiterverwendet oder unter Energiegewinnung verbrannt werden. Das über den Seitenstrom entnommene Wasser ist so sauber, daß es an vielen Stellen in chemischen Prozessen anstelle von vollentsalztem Wasser eingesetzt werden kann.

Das folgende Beispiel dient zur weiteren Erläuterung des erfindungsgemäßen Verfahrens.

Beispiel 1

In eine Glockenbodenkolonne DN 50 mm mit 30 Böden, entsprechend 21 theoretischen Böden, gibt man 2 l/h eines lösungsmittelfreien Reaktionsgemisches aus der DNT-Hydrierung auf den 5. Boden auf. Es handelt sich bei dem Gemisch um eine ca. 60-gew.-%ige Lösung eines Diamingemischs bestehend im wesentlichen aus 77,2 Gew.-% 2,4-Diaminotoluol, 19,3 Gew.-% 2,6-Diaminotoluol und 3,5 Gew.-% anderen Diaminotoluol-Isomeren. Die Lösung weist einen Gehalt an wasserdampfflüchtigen organischen Nebenprodukten von 0,3 Gew.-% auf. Die Kolonne wird unter einem Druck von 3 bar abs. und einer Sumpftemperatur von ca. 200°C betrieben. Am 20. Boden (14. theoretischen Boden) ist ein Entnahmeboden mit Seitenstromentnahme angeordnet. Unter diesem Seitenstrom werden kontinuierlich unter Einhaltung eines Volumenverhältnisses an dieser Stelle der Destillationskolonne von Rücklauf zu Entnahme R/E = 0,4 750 ml/h 133°C heißes, destilliertes Wasser mit einem Gehalt an organischen Nebenprodukten von 200 ppm (Gewicht) entnommen.

Die Brüden der Kolonne werden kondensiert und über einen Phasenabscheider auf den Kopf der Kolonne (30. Boden) zurückgeführt, wobei die Menge der zurückgeführten wässrigen Phase 1050 ml/h und die gleichzeitig dem Phasenscheider kontinuierlich entnommene Menge an organischer Phase 8 ml/h beträgt. Diese organische Phase weist einen Wassergehalt von 4 Gew.-% auf.

Das als Sumpfprodukt kontinuierlich in einer Menge von 1240 ml/h gewonnene Diamin enthält noch einen Restgehalt an Wasser von 3,5 %.

**Patentansprüche**

1. Verfahren zur Aufarbeitung von wässrigen Aminlösungen, wie sie bei der katalytischen Hydrierung von aromatischen Dinitroverbindungen anfallen, und die neben den entsprechenden aromatischen Diaminoverbindungen wasserdampfflüchtige organische Verbindungen als Produkte von Nebenreaktionen enthalten, durch destillative Abtrennung des Wassers zusammen mit den wasserdampfflüchtigen organischen Nebenprodukten in einer Destillationskolonne, dadurch gekennzeichnet, daß man

a) die Brüden der Destillationskolonne kondensiert, die hierbei anfallende flüssige Phase über eine Trennapparatur führt, in welcher dem Brüdenkondensat wasserdampfflüchtige organische Nebenprodukte als organische Phase entnommen werden,

b) die dieTrennapparatur verlassende wässrige Phase auf den Kopf der Destillationskolonne zurückführt,

c) der Destillationskolonne das weitgehend von wasserdampfflüchtigen organischen Verunreinigungen befreite Wasser über einen Seitenstrom entnimmt und

d) die von Wasser und wasserdampfflüchtigen Verunreinigungen weitgehend befreiten Diamine als Sumpfprodukt gewinnt.

2. Verfahren gemäß Anspruch 1, gekennzeichnet dadurch, daß man als Destillationskolonne eine solche mit 12 bis 40 theoretischen Böden verwendet und den Seitenstrom zur Entnahme des Wasser mindestens 4 theoretische Böden unterhalb des Kopfs und mindestens 8 theoretische Böden oberhalb des Sumpfs der Kolonne entnimmt.

3. Verfahren gemäß Anspruch 1 und 2, dadurch gekennzeichnet, daß das Verhältnis von Rücklauf zu Entnahme an der Entnahmestelle des Seitenstroms mindestens bei 0,3 liegt.

4. Verfahren gemäß Anspruch 1 bis 3, dadurch gekennzeichnet, daß man als wäßrige Aminlösungen solche von Diaminotoluolen verwendet, wie sie bei der technischen Hydrierung von technischen Dinitrotoluolen und gegebenenfalls anschließender destillativer Entfernung von gegebenenfalls mitverwendetem Hilfslösungsmittel anfallen.

5. Verfahren gemäß Anspruch 1 bis 4, dadurch gekennzeichnet, daß die Destillationskolonne unter einem Druck von 1,5 bis 6 bar abs. bestrieben wird.

6. Verfahren gemäß Anspruch 5, dadurch gekennzeichnet, daß die Kondensationsenergie der Brüden technisch genutzt wird.

## Claims

1. A process for the working up of aqueous amine solutions as obtained from the catalytic hydrogenation of aromatic dinitro compounds and containing steam volatile organic compounds as products of side reactions in addition to the appropriate aromatic diamino compounds by distillative removal of the water together with the steam volatile organic by-products in a distillation column, characterised in that

a) the vapours from the distillation column are condensed, the liquid phase thus obtained is passed through a separator in which steam volatile organic by-products are removed as organic phase from the vapour condensate,

b) the aqueous phase leaving the separator is returned to the head of the distillation column,

c) the water substantially freed from the steam volatile organic impurities is removed from the distillation column in a side stream and

d) the diamines substantially freed from water and steam volatile impurities are obtained as sump products.

2. Process according to Claim 1, characterised in that the distillation column used is one which has 12 to 40 theoretical shelves and the side stream for the removal of water is discharged at least four theoretical shelves below the head and at least 8 theoretical shelves above the sump of the column.

3. Process according to Claims 1 and 2, characterised in that the ratio of backflow to removal at the discharge point of the side stream is at least 0.3.

4. Process according to Claims 1 to 3, characterised in that the aqueous amine solutions used are solutions of diaminotoluene such as are obtained from the industrial hydrogenation of commercial dinitrotoluene optionally followed by distillative removal of any auxiliary solvents used.

5. Process according to Claims 1 to 4, characterised in that the distillation column is operated under a pressure of from 1.5 to 6 bar abs..

6. Process according to Claim 5, characterised in that the energy of condensation of the vapours is utilized industrially.

## Revendications

1. Procédé de traitement de solutions aqueuses d'amines telles qu'on en obtient dans l'hydrogénation catalytique de composés aromatiques dinitrés, et qui contiennent, à côté des composés aromatiques diaminés correspondants, des composés organiques entraînables par la vapeur d'eau comme produits de réactions secondaires, par séparation par distillation de l'eau conjointement avec les sous-produits organiques entraînables par la vapeur d'eau dans une colonne de distillation, caractérisé en ce que

a) on condense les vapeurs de la colonne de distillation, on transfère la phase liquide ainsi obtenue dans un appareil de séparation dans lequel on soutire du condensat des vapeurs des sous-produits organiques entraînables par la vapeur d'eau, comme phase organique,

b) on recycle la phase aqueuse sortant de l'appareil de séparation à la tête de la colonne de distillation,

c) on soutire la colonne de distillation par un conduit latéral largement débarrassé des impuretés organiques entraînables par la vapeur d'eau et

d) on recueille comme produit de queue les diamines largement débarrassées de l'eau et des impuretés entraînables par la vapeur d'eau.

2. Procédé suivant la revendication 1, caractérisé en ce qu'on utilise comme colonne de distillation, une colonne de 12 à 40 plateaux théoriques et on prélève le courant latéral pour le soutirage de l'eau au moins 4 plateaux théoriques au-dessous de la tête et au moins 8 plateaux théoriques au-dessus de la base de la colonne.

3. Procédé suivant les revendications 1 et 2, caractérisé en ce que le rapport du reflux au volume soutiré à l'endroit de soutirage du courant latéral est au moins égal à 0,3.

4. Procédé suivant les revendications 1 à 3, caractérisé en ce qu'on utilise comme solutions aqueuses d'amines des solutions de diaminotoluènes du type que l'on obtient dans l'hydrogénation industrielle de dinitrotoluènes techniques, suivie le cas échéant de l'élimination par distillation du solvant auxiliaire utilisé simultanément le cas échéant.

5. Procédé suivant les revendications 1 à 4, caractérisé en ce que la colonne de distillation est conduite sous une pression absolue de 1,5 à 6 bars.

6. Procédé suivant la revendication 5, caractérisé en ce que l'énergie de condensation des vapeurs est exploitée techniquement.